(19) Europäisches Patentamt
European Patent Office
Office européen des brevets

(11) Veröffentlichungsnummer: **0 100 498**
**B1**

(12) # EUROPÄISCHE PATENTSCHRIFT

(45) Veröffentlichungstag der Patentschrift: **27.11.85**

(51) Int. Cl.⁴: **C 25 B 3/04,** C 07 C 59/115, C 07 C 103/167, C 07 C 121/36

(21) Anmeldenummer: **83107216.0**

(22) Anmeldetag: **22.07.83**

(54) **Verfahren zur Herstellung von Dichlormilchsäure oder dem Nitril oder Amid der Dichlormilchsäure.**

(30) Priorität: **31.07.82 DE 3228663**

(43) Veröffentlichungstag der Anmeldung: **15.02.84 Patentblatt 84/7**

(45) Bekanntmachung des Hinweises auf die Patenterteilung: **27.11.85 Patentblatt 85/48**

(84) Benannte Vertragsstaaten: **CH DE GB IT LI**

(56) Entgegenhaltungen:
**DD - A - 151 187**

**BEILSTEINS HANDBUCH DER ORGANISCHEN CHEMIE, 4. Auflage, Band 3, 1921, Berlin VERLAG von JULIUS SPRINGER**
**JUSTUS LIEBIG'S ANNALEN DER CHEMIE, Band 179, 1875, Leipzig, Heidelberg PINNER UND BISCHOFF "Reduction der Trichlormilchsäure"**

(73) Patentinhaber: **BASF Aktiengesellschaft, Carl-Bosch-Strasse 38, D-6700 Ludwigshafen (DE)**

(72) Erfinder: **Puetter, Hermann, Dr., Landauer Strasse 59, D-6730 Neustadt (DE)**

**EP 0 100 498 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Diese Erfindung betrifft ein neues Verfahren zur Herstellung von Dichlormilchsäure oder dem Nitril oder Amid der Dichlormilchsäure durch elektrochemische Reduktion der entsprechenden Trichlorverbindungen.

Aus Ann. 257 (1890) 334 ist bekannt, dass man Dichlormilchsäure durch Umsetzung von Dichloracetaldehyd mit Blausäure und anschliessende Verseifung herstellen kann. Dichloracetaldehyd ist teuer und wegen seiner Instabilität nicht beliebig verfügbar. Wegen seiner Unbeständigkeit wird er im allgemeinen in Form seines Diethylacetals gehandelt, d. h. er wird in eine für die Cyanhydridsynthese überflüssige Veredlungsstufe übergeführt.

Es wurde nun gefunden, dass man Dichlormilchsäure oder das Nitril oder Amid der Dichlormilchsäure wirtschaftlich günstiger und technisch erheblich vorteilhafter herstellen kann, wenn man eine Verbindung der Formel:

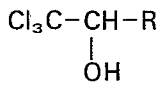 (I)

in der eine NC-, HOOC- oder $H_2NCO$-Gruppe bedeutet, elektrochemisch reduziert.

Nach dem neuen Verfahren erhält man die Dichlormilchsäure oder die Dichlormilchsäurederivate auf technisch einfache Weise und in hoher Selektivität. Dies ist überraschend, da bei der bekannten Reduktion von Trichlormilchsäure mit Hilfe saurer und alkalischer Reduktionsmittel ein Gemisch verschiedener Reduktionsprodukte erhalten wurde. So hat man z. B. festgestellt, dass die bei der Reduktion gebildete Dichlormilchsäure sogar rascher weiterreduziert wird als die Ausgangsverbindung (Ann., *179* (1875) 85). Ausserdem hat sich dabei gezeigt, dass aus Trichlormilchsäure in einer Nebenreaktion Di- und Monochloracrylsäure gebildet werden (Ber. 17 (1884) 2002).

Ausgangsstoffe für das neue Verfahren sind Trichlormilchsäurenitril, Trichlormilchsäureamid oder Trichlormilchsäure. Man erhält dabei als Verfahrensprodukte der elektrochemischen Reduktion das Nitril oder Amid der Dichlormilchsäure bzw. die Dichlormilchsäure.

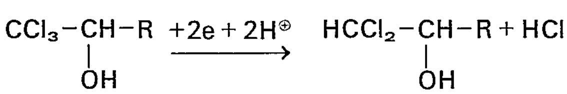


(I) (II)

Das bei der Reduktion von Trichlormilchsäurenitril entstehende Nitril oder Amid der Dichlormilchsäure kann gewünschtenfalls auf an sich bekannte Weise in guter Ausbeute zu Dichlormilchsäure verseift werden. Man kann diese Verseifung auch im Kathodenraum während der Elektrolyse durchführen.

Die erfindungsgemässe Elektrolyse, bei der die Ausgangsstoffe an der Kathode reduziert werden, führt man zweckmässigerweise in einer geteilten Zelle durch. Diese Zellen können monopolar ausgeführt und aus mehreren Modulen zusammengesetzt sein. Man kann als Elektrolysezelle auch eine bipolare Platten- und Rahmenzelle, die auch als Filterpressenzelle bezeichnet wird, verwenden. Als Kathoden nimmt man zweckmässigerweise solche, deren Wasserstoffüberspannung kleienr ist als die Wasserstoffüberspannung an Quecksilber. Beispielsweise sind Feststoffelektroden aus Blei, Eisen, Nickel, Zinn, Stahl, Graphit oder graphitgefüllten Polymeren gut geeignet.

Als Anoden verwendet man für Elektrolysen an sich übliches Anodenmaterial, wie Graphit, Blei oder Titan. Zweckmässigerweise verwendet man Anoden, die dem jeweiligen Anodenprozess angepasst sind. So nimmt man z. B. mit Bleidioxid oder Mangandioxid beschichtete Anoden, wenn diese als Sauerstoffentwickler arbeiten sollen oder Anoden aus Graphit, wenn sie als Chlorentwickler eingesetzt werden sollen. Besonders vorteilhaft ist es, wenn man gleichzeitig Anoden und Kathoden aus Graphit verwendet. Als Trennwand zwischen Anolyt und Katholyt sind Ionenaustauschermembranen gut geeignet. Besonders vorteilhaft sind Kationenaustauschermembranen, da sie eine weitgehende Kontrolle des pH-Wertes ermöglichen. Es ist mit einer solchen Zelle möglich, die bei der Reduktion freiwerdenden $Cl^{\ominus}$-Ionen als HCl im Katholyten anzureichern, wenn man mit einem sauren Anolyten arbeitet. Diese Fahrweise ist deshalb vorteilhaft, weil sie gestattet, bis zum Schluss der Reduktion bei konstanter Stromdichte zu elektrolysieren, ohne dass gegen Ende eine stärkere Überreduktion stattfindet. Überraschend gelangt man so auf hohe Raumzeitausbeuten, da die Stromausbeute fast bis zum Ende der Reduktion konstant und hoch ist.

Der Katholyt enthält die Ausgangsverbindung zweckmässig im gemisch mit Wasser. Vorzugsweise verwendet man ein wässeriges Gemisch, das auf 100 Gewichtsteile Wasser 1 bis 40 Gewichtsteile der Trichlorverbindung enthält. Der Katholyt ist vorzugsweise frei von Hilfsstoffen, da die Ausgangsstoffe ausreichend dissoziieren oder weil der Kathodenraum im Dauerbetrieb hinreichende Mengen an Chlorionen enthält, die bei der Reduktion frei werden. Man kann natürlich auch dissoziierbare Substanzen, wie beispielsweise Salzsäure, Natriumchlorid oder Schwefelsäure zusetzen. Der Anolyt ist frei wählbar; zweckmässigerweise verwendet man eine wässerige Lösung von Natriumchlorid, Schwefelsäure oder Salzsäure. Die Verwendung von sauren Katholyten bei einer mit einer Kationenaustauschermembrane geteilten Zelle ist aus dem oben dargelegten Grunde vorteilhaft.

Man elektrolysiert zweckmässig bei Temperaturen bis 70°C, vorzugsweise zwischen 5 und 30°C und bei Stromdichten zwischen 0,1 und 20 $A/dm^2$, vorzugsweise zwischen 1 und 10 $A/dm^2$. Es ist nicht erforderlich, die Stromdichte im Laufe der Reduktion zu regeln, beispielsweise, indem man ein konstantes Elektrodenpotential einhält. Man kann also auf technisch besonders einfache Weise bei konstanter Stromdicht

arbeiten. Es ist vorteilhaft, wenn der pH-Wert des Katholyten unter 6, vorzugsweise unter 4 liegt. Besonders günstig ist der Bereich von 0 bis 1,5.

Die Wasserstoffbildung ist die bevorzugte kathodische Nebenreaktion. Die während der Reduktion freiwerdende Wasserstoffmenge liegt zwischen < 0,1 mol/F und 0,5 mol/F. Es hat sich zur Erlangung hoher Umsätze als günstig erwiesen, die Elektrolyse so lange bei konstanter Stromdichte durchzuführen, bis die Wasserstoffentwicklung auf einen Wert zwischen 0,1 und 0,3 mol/F angestiegen ist, und dann die Elektrolyse abzubrechen. Es werden auch bei hohen Umsätzen (> 95%) und hohen Stromdichten (> 3A), d. h. bei hohen Raumzeitausbeuten Selektivitäten von 90% und mehr erreicht.

Dass man bei der Reduktion hohe Umsätze erzielt und dabei weder eine Weiterreduktion noch durch Säuren katalysierte Nebenreaktionen beobachtet, ist überraschend, da sich der Säuregehalt im Katholyten bei Verwendung saurer Anolyte im Laufe der Elektrolyse erhöht, und die Reaktion dabei in Richtung einer Wasserstoffentwicklung verschoben wird. Es ist auch überraschend, dass man das vorteilhafte Ergebnis bei Verwendung von wässerigen Gemischen der Ausgangsstoffe und an Kathoden mit mittlerer Wasserstoffüberspannung erhält, da sich aliphatische Halogenverbindungen in Wasser nur sehr schwer reduzieren lassen und die meisten kathodischen Enthalogenierungen, besonders im wässerigen Milieu, wenn überhaupt nur an Elektroden mit höchster Wasserstoffüberspannung, nämlich Hg-Elektroden, gelingen.

Nach dem erfindungsgemässen Verfahren lassen sich Dichlormilchsäure bzw. die Dichlormilchsäurederivate technisch einfach und besonders wirtschaftlich herstellen. Dichlormilchsäure wird als Zwischenprodukt für Farbstoffe, Pflanzenschutzmittel und Pharmaka benötigt. Dichlormilchsäurenitril ist ausserdem ein stabiles, lagerfähiges Vorprodukt für Dichloracetaldehyd, der seinerseits ein wertvolles Zwischenprodukt z. B. für die Herstellung von Insektiziden darstellt. Dichlormilchsäureamid kann als Zwischenprodukt für die Herstellung von Heterocyclen verwendet werden.

*Beispiel 1:*

Man verwendet eine Elektrolyseapparatur, die aus einer Platten- und Rahmenzelle mit einer Graphitplatte als Anode (aktive Fläche: 1 $dm^2$), einer Bleiplatte als Kathode (aktive Fläche: 1 $dm^2$), zwei rechteckigen PVC-Rahmen (Innenmasse; 7,1 × 14,0 cm ≙ 1 $dm^2$, Dicke: 1 cm) und einer zwischen den Rahmen angebrachten Kationenaustauschermembran (aus sulfonierten Fluorpolymeren) besteht. Die Apparatur ist ausserdem mit zwei Elektrolytkreisläufen versehen, die mit je einem Glaskühler, einem 2-l-Vorratsgefäss aus Glas und einer Magnetkreiselpumpe ausgerüstet ist.

Als Anolyt verwendet man 1,5 kg einer 5%ig. Salzsäure. Der Katholyt besteht aus 174 g (0,9 mol) Trichlormilchsäure und 826 g Wasser. Der pH des Katholyten beträgt 1,1.

Man elektrolysiert bei einer Stromstärke von 3 A (Stromdichte 3 A/$dm^2$), einer Temperatur im Anolyten von 10° C und einer Temperatur im Katholyten von 7° C. Anolyt und Katholyt werden dabei umgepumpt (ca. 150 l/h). Man elektrolysiert so lange bei konstanter Stromstärke, bis die Wasserstoffentwicklung 0,15 mol $H_2$/F überschritten hat; dies ist nach 19 h der Fall. Die Elektrolyse wird abgebrochen, der Katholyt wird mit Natriumchlorid versetzt und mit Methyl-tertiär-butylether mehrfach extrahiert. Die getrockneten organischen Phasen werden im Rotationsverdampfer eingedampft.

Man erhält 135,2 g Verfahrensprodukt in Form weisser Kristalle (Materialausbeute 85%, Stromausbeute 72%). Das Produkt mit der Zusammensetzung 95% Dichlormilchsäure, 3% Trichlormilchsäure und 2% Monochlormilchsäure hat einen Schmelzpunkt von 70 bis 72° C.

Durch Umkristallisation aus Toluol werden 111,5 g reine Dichlormilchsäure mit dem Schmelzpunkt 78 bis 79° C erhalten (Ausbeute 78%).

*Elementaranalyse:*

Berechnet: C 22,7 H 2,5O 30,2 Cl 44,6%

Gefunden: C 22,7 H 2,6O 30,3 Cl 44,4%

*Beispiele 2:*

Man elektrolysiert unter Verwendung einer Pb/$PbO_2$-Platte als Anode in der in Beispiel 1 beschriebenen Apparatur. Der Anolyt besteht aus 1,5 kg 10%ig. Schwefelsäure, der Katholyt aus 96,8 g (0,5 mol) Trichlormilchsäure und 800 g Wasser. Der pH beträgt 1,4.

Bei einer konstanten Stromstärke von 5 A wird 6,25 h elektrolysiert. Die Elektrolysetemperatur betägt 15° C. Insgesamt sind 4,4 l Wasserstoff entstanden (8% d. Th.), die Bildungsgeschwindigkeit betrug zuletzt 0,26 mol $H_2$/F.

Der Anolyt wird nach Neutralisation der freien Mineralsäuren (HCl, $H_2SO_4$) durch Zugabe von Natronlauge im Rotationsverdampfer eingedampft. Der Rückstand wird in Methylenchlorid aufgenommen und die Methylenchlorid-Phase eingedampft. Man isoliert als hellen Festkörper 57 g leicht verunreinigte Dichlormilchsäure (Ausbeute 72%). Durch Umkristallisation aus Toluol, erhält man weisse Kristalle von Schmelzpunkt 74 bis 76° C (Reinheit 97%).

*Beispiel 3:*

Man verfährt wie in Beispiel 2 beschrieben, wobei man den zunächst anfallenden Festkörper nicht mit Methylenchlorid, sondern mit Methyl-tertiär-butylether extrahiert. Rohausbeute: 65,7 g ≙ 83% d. Th. (Gehalt ⩾ 90% Dichlormilchsäure, Tri- und Monochlormilchsäure je ⩽ 5%). Nach Umkristallisation aus Toluol erhält man weisse Kristalle vom Schmelzpunkt 74 bis 76° C. Ausbeute der Kristallisation: 78%.

*Beispiel 4:*

Man elektrolysiert unter Verwendung von Anoden und Kathoden aus Graphit in der in Beispiel 1 beschriebenen Apparatur. Der Katholyt besteht

aus 800 g Wasser und 96,8 g Trichlormilchsäure, der Anolyt aus 1,5 kg 5%ig. Salzsäure.

Bei einer Temperatur um 10°C wird mit einer Stromdichte von 5 $A/dm^2$ 10 h elektrolysiert. Am Ende beträgt die Strommenge 187% der Theorie und die Wasserstoffentwicklung ~ 0,4 mol $H_2/F$. Der Katholyt wird mit Natriumchlorid ausgesalzen und mit Methyl-tertiär-butylether extrahiert. Zurück bleibt ein langsam kristallisierendes Öl, es enthält 75% Dichlormilchsäure und 25% Trichlormilchsäure. Monochlormilchsäure wird nicht gefunden.

*Beispiel 5:*

Man verfährt wie in Beispiel 4 beschrieben, wobei man als Katholyt ein Gemisch aus 174,5 g (1 mol) Trichlormilchsäurenitril und 825 g 1%ig. Salzsäure und als Anolyt 2 l 5%ig. Salzsäure verwendet. Der pH des Katholyten beträgt 0,6.

Bei einer Stromdichte von 3 $A/dm^2$ und einer Elektrolysetemperatur von 10°C wird 18 h elektrolysiert. Der Ansatz wird mit Methyl-tertiär-butylether mehrfach extrahiert, die organischen Lösungen werden getrocknet und eingedampft. Man erhält eine Rohausbeute von 91%, der Gehalt an Monochlormilchsäurenitril beträgt etwa 2% und der Gehalt an Trichlormilchsäurenitril < 2%.

*Beispiele 6 bis 9:*

Unter Verwendung der in Beispiel 1 bechriebenen Apparatur und des in der Tabelle angegebenen Kathodenmaterials wird bei 13°C und einer konstanten Stromstärke von 5 A bis zu einer Strommenge von 54 Ah elektrolysiert. Der Katholyt besteht aus 174,4 g (1 mol) Trichlormilchsäurenitril und 1 kg 1%ig. Schwefelsäure und der Anolyt aus 1 kg 10%ig. Schwefelsäure. Der pH des Katholyten beträgt 0,7.

Man erhält dabei die in der Tabelle genannten Ergebnisse:

*Tabelle*

| Beispiel Nr. | Elektroden-material | Umsatz (%) | Produktzusammensetzung | | Ausbeute (g) |
|---|---|---|---|---|---|
| | | | Dichlor- | Monochlor- | |
| 6 | Pb | 75 | 93% | 7% | 141 |
| 7 | Sn | 35 | 100% | — | 161 |
| 8 | Ni | 55 | 100% | — | 150 |
| 9 | R4A | 50 | 100% | — | 145 |

*Beispiel 10:*

Dieses Beispiel soll zeigen, dass die elektrolytische Reduktion auch bei höheren Strommengen von hoher Selektivität ist.

Man elektrolysiert in der in Beispiel 1 beschriebenen Apparatur 0,5 mol Trichlormilchsäure in 800 g $H_2O$ über 19 h bei einer Stromstärke von 6 A (6 $A/dm^2$). Nach 5 h ist die Wasserstoffentwicklung auf 32% der theoretischen Menge (0,5 mol $H_2/F$) angestiegen. Die Strommenge beträgt nach 5 h 112% d. Th., der Umsatz ca. 90%. Man setzt die Elektrolyse weitere 14 h fort, bis eine Strommenge von insgesamt 425% erreicht ist. Der Katholyt wird mit Methyl-tertiär-butylether mehrfach extrahiert, die Lösungsmittel-Extrakte werden eingeengt. Man erhält 58,9 g weisse Kristalle vom Schmelzpunkt 70°C. Die Rohausbeute beträgt 74%. Das Rohprodukt enthält 90% Dichlormilchsäure und 10% Monochlormilchsäure.

*Beispiel 11:*

Man elektrolysiert unter Verwendung von Anoden und Kathoden aus Graphit in der in Beispiel 1 beschriebenen Apparatur.

Der Katholyt besteht aus 96,2 g (0,5 mol). Trichlormilchsäureamid, 973 g Wasser und 27 g 37%ig. Salzsäure. Der pH beträgt 0,7. Der Anolyt besteht aus 1,5 kg 5%ig. Salzsäure.

Bei einer Temperatur von 21°C wird 15 h bei 2 A elektrolysiert (≙ 112% der theoretischen Strommenge). Nach dieser Zeit ist der Umsatz 100% (gemäss einer NMR-Probe direkt aus dem Elektrolyten).

Der Katholyt wird auf pH 7 gestellt, mit Natriumchlorid ausgesalzen und mit Methyl-tertiär-butylether 24 h kontinuierlich extrahiert.

Nach Trocknen und Einrotieren der organischen Phase erhält man 69,8 g (Rohausbeute: 88%) eines hellgelben Öls, das allmählich erstarrt. Es enthält zu 95% Dichlormilchsäureamid, daneben Spuren von Ausgangsmaterial Wasser und Lösungsmittel. Monochlormilchsäureamid wird nicht gefunden. Umkristallisation aus Tetrahydrofuran ergibt reines Dichlormilchsäureamid, Fp 77 bis 78°C. Ausbeute der Umkristallisation 70%.

## Patentansprüche

1. Verfahren zur Herstellung von Dichlormilchsäure oder dem Nitril oder Amid der Dichlormilchsäure, dadurch gekennzeichnet, dass man eine Verbindung der Formel:

$Cl_3C-CH-R$ (I)
|
OH

in der R eine NC-, HOOC- oder $H_2NCO$-Gruppe bedeutet, elektrochemisch reduziert.

2. Verfahren nach Anspruch 1, dadurch gekennzeichnet, dass man die Reduktion in einer geteilten Zelle durchführt.

3. Verfahren nach Anspruch 1, dadurch gekennzeichnet, dass man die Reduktion in einer durch Kationenaustauschermembranen geteilten Zelle durchführt.

4. Verfahren nach Anspruch 1, dadurch gekennzeichnet, dass man saure Anolyten verwendet.

5. Verfahren nach Anspruch 1, dadurch gekennzeichnet, dass der pH-Wert des Katholyen während der elektrolytischen Reduktion unter 6 liegt.

6. Verfahren nach Anspruch 1, dadurch gekennzeichnet, dass man die elektrolytische Reduktion bis zu einer Wasserstoffentwicklung von 0,1 mol/F bis 0,3 mol/F durchführt.

## Revendications

1. Procédé de préparation de l'acide dichlorolactique ou du nitrile ou de l'amide de l'acide dichloro-lactique, caractérisé en ce que l'on soumet un composé de la formule:

$Cl_3C-CH-R$ (I)
|
OH

dans laquelle R désigne un groupe NC-, HOOC- ou $H_2NCO$-, à une réduction électrochimique.

2. Procédé suivant la revendication 1, caractérisé en ce que la réduction est réalisée dans une cellule divisée.

3. Procédé suivant la revendication 1, caractérisé en ce que la réduction est réalisée dans une cellule divisée par des membranes échangeuses de cations.

4. Procédé suivant la revendication 1, caractérisé en ce que l'on emploie des anolytes acides.

5. Procédé suivant la revendication 1, caractérisé en ce que, pendant la réduction, le pH du catholyte est maintenu à une valeur inférieure à 6.

6. Procédé suivant la revendication 1, caractérisé en ce que la réduction est poursuivie jusqu'à une production d'hydrogène comprise entre 0,1 mol/F et 0,3 mol/F.

## Claims

1. A process for the preparation of dichlorolactic acid or the nitrile or amide thereof, wherein a compound of the formula:

$Cl_3C-CH-R$ (I)
|
OH

where R is an NC, HOOC or $H_2NCO$ group, is reduced electrochemically.

2. A process as claimed in Claim 1, wherein the reduction is carried out in a divided cell.

3. A process as claimed in Claim 1, wherein the reduction is carried out in a cell divided by a cation exchanger membrane.

4. A process as claimed in Claim 1, wherein an acid anolyte is employed.

5. A process as claimed in Claim 1, wherein the pH of the catholyte during the electrolytic reduction is below 6.

6. A process as claimed in Claim 1, wherein the electrolytic reduction is carried out until the amount of hydrogen evolved is from 0.1 mole/F to 0.3 mole/F.